(19) Europäisches Patentamt / European Patent Office / Office européen des brevets

(11) **EP 3 442 403 B1**

(12) **EUROPEAN PATENT SPECIFICATION**

(45) Date of publication and mention
of the grant of the patent:
**09.10.2019 Bulletin 2019/41**

(51) Int Cl.:
*A61B 5/00* *(2006.01)*     *A61B 5/11* *(2006.01)*
*G06K 9/00* *(2006.01)*     *G06K 9/62* *(2006.01)*

(21) Application number: **17751723.2**

(22) Date of filing: **09.08.2017**

(86) International application number:
**PCT/EP2017/070245**

(87) International publication number:
**WO 2018/029275 (15.02.2018 Gazette 2018/07)**

(54) **PROCESSING APPARATUS AND METHOD FOR DETERMINING AN AMBULATION MOTION OF A SUBJECT**

VERARBEITUNGSVORRICHTUNG UND VERFAHREN ZUR BESTIMMUNG EINER GEHBEWEGUNG EINES PATIENTEN

APPAREIL DE TRAITEMENT ET PROCÉDÉ POUR DÉTERMINER UN MOUVEMENT AMBULATOIRE D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2016 EP 16183332**

(43) Date of publication of application:
**20.02.2019 Bulletin 2019/08**

(73) Proprietor: **Koninklijke Philips N.V.**
**5656 AE Eindhoven (NL)**

(72) Inventors:
• **DERKX, Rene, Martinus, Maria**
**5656 AE Eindhoven (NL)**
• **BLOEMEMDAL, Brian, Brand, Antonius, Johannes**
**5656 AE Eindhoven (NL)**
• **AARTS, Vincent, Alexander, Rudolf**
**5656 AE Eindhoven (NL)**

(74) Representative: **Claveau, Olivier**
**Philips Intellectual Property & Standards**
**High Tech Campus 5**
**5656 AE Eindhoven (NL)**

(56) References cited:
**US-A1- 2008 082 018     US-A1- 2008 190 202**
**US-A1- 2013 298 636     US-A1- 2015 272 480**

• **WANG YUN-JIA ET AL: "Extraction of spine wave from walking acceleration using blind source separation", ZHONGGUO GUANXING JISHU XUEBAO - JOURNAL OF CHINESE INERTIAL TECHNOLOGY, ZHONGGUO CHUANBO ZHONGGONG JITUAN GONGSI. DI-707 YANJIUSUO, CN, vol. 22, no. 4, 31 July 2014 (2014-07-31), pages 426-431, XP009501069, ISSN: 1005-6734**

# Description

## FIELD OF THE INVENTION

The present invention relates to a processing apparatus, system and method for determining an ambulation motion of a subject. The present invention further relates to a corresponding computer program for carrying out said method.

## BACKGROUND OF THE INVENTION

Vital signs of a person, for example the heart rate (HR), can serve as indicators of the current state of a person and as powerful predictors or serious medical events. In a clinical setting, vital signs are generally measured under well-controlled conditions. A measuring device is applied with correct orientation and at a predetermined location by a trained professional. Further, the vital signs are measured in a known context, for example when the patient is well rested or performing a predetermined exercise, for example in an exercise electrocardiography as instructed by a nurse.

With the advent of smaller, cheaper and less obtrusive devices, monitoring a health-status is also becoming feasible at home or in other non-clinical settings. A problem in vital signs measurement is that proper interpretation of vital signs requires context information about what the patient is doing and/or has been doing that could potentially influence the vital sign. Moreover, if context information is available, additional information can be obtained such as (a) monitoring vital signs of a subject at home in a relaxed or resting situation for obtaining baseline vital signs at rest or (b) monitoring vital signs just after a specific activity, for example, recovery of the vital sign after climbing stairs.

For elderly or sick persons, one of the most energy demanding and often occurring activity types is ambulation or walking. It would thus be advantageous to detect walking in an accurate way, in particular for the exemplary medical scenario with elderly and/or sick patients who might walk very slowly.

US 2014/0019080 A1 relates to calibration of a chest-mounted wireless sensor device for posture and activity detection. Therein, a walking detection algorithm is proposed comprising: retrieving raw accelerometer data in three axes (x, y, z), computing a signal magnitude area (SMA) for a predetermined time window, computing a magnitude of acceleration in a horizontal plane ($mag_{horz}$) and overall ($mag_{total}$), and comparing the calculated SMA and magnitude of accelerations to various thresholds. Based on the comparison of said acceleration magnitudes to a specific sequence of different thresholds, the activity can be classified as walking. For calculating the magnitude of acceleration in the horizontal plane, a gravity component is determined as a first step. Based thereon, a magnitude of acceleration in the horizontal plane is calculated as a subsequent step.

US 2008/0190202 describes a motion sensing apparatus comprising an accelerometer and a processing system adapted to analyze acceleration measurements from the accelerometer. In particular, the processing system determines an orientation of the accelerometer to thereby determine an activity of a user.

US 2015/0272480 describes an acceleration sensor output processing program adapted to extract a gravity component vector and a swing component vector from an output of an acceleration sensor which has been passed through a low-pass filter and a high-pass filter respectively.

US 2013/0298636 describes a method of calibrating a posture sensor. In particular, a characteristic of an AC component of an acceleration signal is extracted to determine a walking or non-walking state of a user.

US 2008/0082018 relates to methods of processing respiratory signals. In one example, accelerometer data is used to enhance motion artifact rejection.

Wang Yun-jia et al., Journal of Chinese Inertial Technology vol. 22, no. 4, pp. 426-431 (2014) discloses using blind source separation for gait detection from accelerometer data.

## SUMMARY OF THE INVENTION

It is an object of the present invention to provide an improved device, system and method for determining an ambulation motion of a subject. In particular, it would be advantageous to determine an ambulation motion in an accurate way, even when the subject is walking very slowly.

In a first aspect of the present invention a processing apparatus for determining an ambulation motion of a subject is presented. The processing apparatus is configured to perform the steps of:

> obtaining accelerometer data indicative of a trunk motion of the subject,
> performing a high pass filter on the accelerometer data to obtain high-pass filtered accelerometer data;
> extracting at least one of a plurality of orthogonal motion components from the high-pass filtered accelerometer data;
> performing a low-pass filter on the accelerometer data to obtain low-pass filtered accelerometer data;
> determining a gravity component from the low-pass filtered accelerometer data;
> comparing a direction of at least one of said orthogonal motion components with a direction of the gravity component; and
> classifying a motion of the subject as ambulation if said motion component is orthogonal or parallel to said gravity component.

In a further aspect of the present invention, a system for determining an ambulation motion of a subject is presented, said system comprising:

- an accelerometer for acquisition of accelerometer data indicative of a trunk motion the subject; and
- a processing apparatus as disclosed herein for determining an ambulation motion of the subject.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The herein presented solutions provide a possibility to reliably determine an ambulation motion of the subject, for example, detecting whether the subject is walking. The proposed solution thereby enables obtaining context information that can be used for vital signs measurements in particular for elderly and/or sick persons in a home or out-patient care setting. Since ambulation activities can be very energy demanding for these persons, they may walk slowly and it is very important to provide a reliable way of detecting slow ambulation in order to interpret the vital signs that are being measured correctly. Furthermore, a reliable detection of slow ambulation enables a way for auto-calibration based on distinct motion components also in such scenarios.

On an abstract level, accelerometer data is provided as an input to the proposed processing apparatus and, based thereon, an indication whether the subject is performing an ambulation motion can be provided as an output. The accelerometer data can be acquired by an accelerometer applied to a trunk of the subject. The accelerometer data can in particular refer to tri-axial accelerometer data acquired by a tri-axial accelerometer.

The present invention is based on the idea of extracting at least one of different orthogonal motion components from high-pass filtered accelerometer data (only) and then comparing a direction of at least one of them to a direction of the gravity component. In particular, the orthogonal motion components are extracted using a source-separation technique, which advantageously allows identification of such motion components without the need for knowledge of a direction of gravity. This allows for improved precision and accuracy in identifying and extracting a motion component from the accelerometer data. Moreover, use of a source-separation technique in itself provides a more accurate extraction of motion components from the accelerometer data.

In some embodiments, the method comprises extracting a plurality (i.e. two or more) of orthogonal motion components. In yet further embodiments, the method comprises extracting three orthogonal motion components. The motion components are orthogonal to one another.

Three distinct orthogonal motion components can be identified (1) a medio-lateral motion component (ML), i.e., a sideways or left-to-right motion component; (2) an anterior-posterior motion component (AP), i.e. a forward-backward or front-to-back motion component; and (3) a vertical motion component (VT), i.e., an up-down motion component. It has been found that orthogonal motion components can be extracted based on their different temporal structures. The orthogonal motion components are extracted from high-pass filtered accelerometer data. The use of a high-pass filter leads to accelerometer data which is free from an average gravity vector or gravity component. Hence, the extraction of at least one orthogonal motion component is not biased based on prior knowledge of the gravity component. Instead, a signal which is free from the average gravity component can be used as the basis. Hence, in contrast to solutions as described in a prior art, the coordinate system for the orthogonal motion components is not derived starting from the gravity component. The gravity component is rather used for subsequent comparison.

The gravity component can be determined from low-pass filtered accelerometer data. Such low-pass filtered accelerometer data can be seen as an average contribution of gravity on the accelerometer axes. In the solution proposed herein, the gravity component can then be used for comparison of a direction of at least one of the orthogonal motion components with a direction of the gravity component. Based thereon, a motion of the subject can be classified as ambulation if said motion component is orthogonal or parallel to said gravity component. A medio-lateral motion component and an anterior-posterior motion component are expected to be orthogonal to the gravity component, whereas a vertical motion component is expected to be parallel to or in line with the gravity component.

An advantage of the solution proposed herein is that advantageously also slow ambulation motions of a subject can be detected reliably. In contrast, while well-established for detection of activities such as running, a comparison of acceleration magnitudes to thresholds may fail when evaluating the slow motions of elderly and/or sick persons. In particular the ML component can provide a very good detection quality for slow walking cases. As a further advantage it becomes possible to detect left vs right footsteps. For classifying a motion as ambulation the ML direction can be compared with the gravity direction to see if they are orthogonal.

According to an embodiment, the processing apparatus can be configured to extract a medio-lateral and/or an anterior-posterior motion component from said high-pass filtered accelerometer data. In the alternative or in addition, a vertical motion component can be extracted based on said high-pass filtered accelerometer data.

In an embodiment, the processing apparatus is configured to extract at least one of said plurality of orthogonal motion components based on a source-separation technique, in particular based on linear predictability, blind source separation (BSS) and/or independent component analysis (ICA). Based on such techniques, orthogonal motion components can be determined from high-pass filtered accelerometer data, hence, without using a-priori knowledge of the gravity vector. In consequence, one or more of these extracted orthogonal motion components can be compared with a direction of the gravity component to evaluate whether a characteristic orientation of the respective orthogonal motion component can be found with respect to the gravity component. As an output of a source-separation technique, a respective axis of the at least one orthogonal motion component can be provided and optionally also a signal component along the axis. Memory efficient and cost effective iterative structures for source separation are, for example, described in Choi et al., "Blind Source Separation and Independent Component Analysis: A Review", Neural Information Processing - Letters and Reviews, Vol 6., No. 1, January 2005.

In a refinement, the source-separation technique can be adapted to separate at least one of said orthogonal components based on their temporal structure, in particular by maximizing an auto-correlation at a predetermined time lag. The auto-correlation can also refer to a normalized auto-correlation, e.g., wherein the auto-correlation function at lag zero has been normalized to unity. A time lag as used herein can also refer to a time lag period. In other words, instead of just looking at one lag, an optionally weighted average of multiple lags can be considered which may further improve reliability. It has been found that in particular the direction of a medio-lateral (ML) motion component in arbitrarily oriented high-pass filtered accelerometer data can be determined based on an auto-correlation at a specific time lag. In particular, it has been found that the auto-correlation at a specific lag can be highest for the ML component. Thereby, it is possible to distinguish the ML component e.g. from the VT or AP component. In addition or in the alternative, an AP component can be distinguished from the VT component.

In a refinement, the source separation technique can be adapted to maximize an auto-correlation at a predetermined time lag, wherein said the time lag can be between 100 ms and 500 ms, preferably between 150 ms and 400 ms, preferably between 200 ms and 350 ms. It is to be understood that those value ranges include the boundaries. It has been found that in particular maximizing an auto-correlation and a time lag in the range of 200 ms to 350 ms can be used to determine the medio-lateral component.

In an embodiment, the processing apparatus can be configured to extract a first orthogonal motion component based on a source-separation technique, wherein said source-separation technique is adapted to maximize an auto-correlation at a first predetermined time lag; and further configured to extract a second orthogonal motion component based on a source-separation technique, wherein said source-separation technique adapted to maximize an auto-correlation at a second predetermined time lag. For example, in a first step a medio-lateral motion component can be extracted as the first orthogonal motion component. An appropriate time lag for the auto-correlation maximized by said source-separation technique can be in the range of 200 ms to 350 ms. Once the medio-lateral component has been determined, a sub-space orthogonal to said medio-lateral component can be calculated. In a second step a second orthogonal motion component such as an anterior-posterior motion component can be extracted based on a source-separation technique which may, for example, maximize an auto-correlation at a second predetermined time lag. For example, an auto-correlation at time lag in a range of 50 ms to 100 ms, preferably at 75 ms, can be maximized to obtain the anterior-posterior motion component.

In an embodiment, the step of extracting one or more orthogonal motion components can comprise: extracting a first motion component based on a source separation technique; determining, based on the high-pass filtered accelerometer data and said first motion component, a two-dimensional sub-space orthogonal to said first motion component; and extracting a second motion component based on said sub-space. For example, a medio-lateral motion component can be extracted based on a source-separation technique. In a subsequent step, a second orthogonal motion component such as the anterior-posterior motion component can be extracted from said sub-space. An advantage of using the sub-space is that the dimension can be reduced to a mixture of 2 sources. Hence, the complexity and computational effort can be reduced. This can also be referred to as deflation. Hence, a three source mixture of initial accelerometer data obtained with a tri-axial accelerometer can be reduced to a mixture of two sources. A similar source-separation technique can be applied to extract the second source. The third source, which is orthogonal to the other two sources, is then remaining.

In a refinement of this embodiment, the processing apparatus can be configured to extract said second motion component based on said sub-space and a high-pass filtered norm of the accelerometer data. Hence, as an alternative or in addition to extracting the second orthogonal motion component based on a source-separation technique, an alternative approach can be used. It is thus proposed to exploit the high-pass filtered (vector) norm of the accelerometer data as being a reference signal for the extraction of the vertical motion component from the accelerometer data. An NLMS (normalized least mean squares) technique can be used to find a correlation between the high-pass filtered norm with the high-pass filtered accelerometer data.

For example, after having extracted the vertical component by using the NLMS, a cross-product between this vertical component and the previously determined medio-lateral component can be computed to extract the anterior-posterior component. One or more of those orthogonal motion components can then be compared with the direction of the gravity component. For example, if the medio-lateral component and/or the anterior-posterior component are orthogonal to the gravity component, the motion of the subject can be classified as ambulation.

In an embodiment, in the step of classifying a motion of the subject as ambulation the motion component can be deemed to be orthogonal to the gravity component if an absolute value of a dot product of the motion component and the gravity component is below a predetermined threshold. It shall be understood that a motion component and/or gravity component as used herein can refer to a normalized vector or value. Further, it shall be understood that for the ML and AP orthogonal motion components the dot product with the gravity component will ideally be zero. However, as used herein, these motion components and the gravity component are considered to be orthogonal if an absolute value of a dot product of the motion component and the gravity component is below a predetermined threshold. For example, an angular deviation of up to $\pm 10°$ in particular up to $\pm 5°$ may still be accepted. Correspondingly, in the step of classifying a motion of the subject as ambulation the VT motion component can be deemed to be parallel to the gravity component if an absolute value of a vector product or cross product of the VT motion component and the gravity component is below a predetermined threshold. It shall be understood that this also includes the equivalent case that instead of a cross-product of two parallel vectors being zero, the property that the absolute value of the dot-product of two normalized and parallel vectors is close to unity can be evaluated.

In an embodiment, the processing apparatus can further be configured to perform the step of calibrating the accelerometer data based on the at least one extracted orthogonal motion component. For example, the axes of the orthogonal motion components ML, AP, VT can be used to perform auto-calibration of an accelerometer. An advantage of this embodiment is that it can enable improved posture detection in case people are free to apply the accelerometer on the trunk in different positions. Hence, an orientation of an accelerometer applied by a lay-person can be corrected for advantageously also in case of a slow-moving elderly and/or sick subject.

In an embodiment the processing apparatus can further be configured to perform the step of identifying individual steps and/or identifying a gait parameter during ambulation based on the at least one the extracted orthogonal motion component. For example, a left-right movement in the medio-lateral motion component can be evaluated to determine individual steps. In addition or in the alternative to step detection, gait-related parameters can advantageously be derived since they can be clinically relevant in particular for deterioration detection.

In a refinement, the processing apparatus can be configured to identify individual left and right steps based on rising and falling zero-crossings of a medio-lateral motion component and/or based on valleys in a vertical or anterior-posterior motion component. For example, said valleys can be indicative of a toe-off moment of a step. An identification of individual left and right steps further allows computing an accurate step count.

As used herein, the term "ambulation" or ambulation motion can generally refer to walking or a walking motion. The term can also encompass a broader range of walk-like activities such as climbing stairs, walking at different speeds, walking irregular or asymmetric, an/or with the used of walking aids such as a stick, cane, walker or crutch.

The term "accelerometer data" as used herein can refer to accelerometer data in particular 3D or tri-axial accelerometer data being acquired by a tri-axial accelerometer applied to a trunk of the subject. A tri-axial accelerometer is a device that measures the acceleration in three directions referred to as sensing axes $x, y,$ and $z$. A reading of the accelerometer can comprise a three element vector having an $x$-, $y$-, and $z$-component representing the acceleration measured on the respective sensing axes. The accelerometer data can represent a series of such vectors over time. The accelerometer axes are not necessarily coincident with the body axis of a subject since the accelerometer may be applied to the trunk of the subject in an arbitrary orientation and not perfectly defined position. Hence, there is a need to extract orthogonal motion components indicative of the different body axis. A rotation matrix can be applied to align the axis of the accelerometer with the body axis.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a system according to an aspect of the present invention;
Fig. 2 shows a flow chart of a method according to an aspect of the present invention;
Fig. 3 shows a schematic diagram of a second embodiment of a system according to an aspect of the present invention;
Figs. 4A and 4B show diagrams illustrating accelerometer data acquired by a tri-axial accelerometer applied to a trunk of a subject and aligned with the body axes of the subject as well as the vector norm for a fast walking subject and a slow walking subject, respectively;

Fig. 5 shows exemplary diagrams of an autocorrelation for different orthogonal motion components for different types of ambulation;

Figs. 6A and 6B show diagrams illustrating accelerometer data acquired by a tri-axial accelerometer applied to a trunk of a subject but not aligned with the body axes of the subject as well as the vector norm for fast walking and slow walking, respectively;

Figs. 7A and 7B show a diagram illustrating signals based on extracted motion components from high-pass filtered accelerometer data for fast walking and slow walking, respectively;

Fig. 8 shows diagrams of extracted orthogonal motion components;

Fig. 9 shows a schematic diagram of a further embodiment of the device according to an aspect of the present invention; and

Fig. 10 shows a schematic diagram of another embodiment of the device according to an aspect of the present invention.

## DETAILED DESCRIPTION OF THE EMBODIMENTS

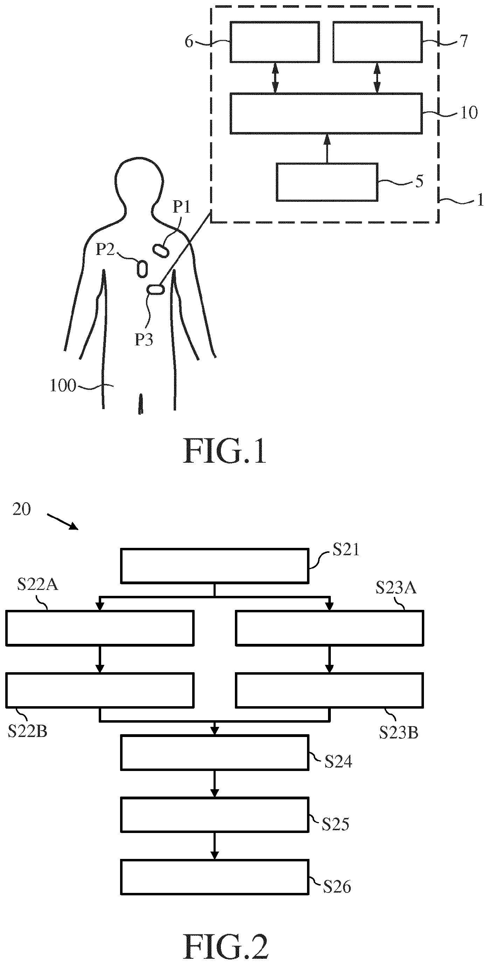

Fig. 1 schematically shows a first embodiment of a system 1 for determining an ambulation motion of a subject 100 according to an aspect of the present invention. The system 1 comprises an accelerometer 5 adapted to obtain accelerometer data indicative of a trunk motion of the subject 100. Based thereon, the processing apparatus 10 can determine an ambulation motion of a subject 100.

As shown in the non-limiting example of Fig. 1, the system 1 can be applied at various positions PI, P2, P3 on the torso or trunk of the subject 100. The exact orientation and position where the subject 100 applies the system 1 may vary, in particular because the device is not necessarily applied by a trained healthcare professional but may be applied by the subject himself. In other words, the application or attachment of the system 1 comprising the accelerometer 5 may be done in an unsupervised manner. The subject 100 may place the system onto several places on the trunk such as on the chest, hip or lower back and may even choose a different location from day to day. In case of combined monitoring of ECG and ambulation motion a different location, e.g., PI, P2 or P3, may be chosen from day to day to avoid or reduce skin irritation if the device is worn over an extended period of time. This means that it cannot always be assumed a priori that the system is at a specific location in a known orientation.

The accelerometer 5 can be implemented as a multi-axial accelerometer adapted to generate accelerometer data indicative of the acceleration along different spatial axes, in the following a tri-axial accelerometer adapted to generate accelerometer data indicative of the acceleration along three orthogonal spatial axes. Exemplary three-axial accelerometers are the Bosch BMA355, ST Microelectronics LIS3DSH, ST Microelectronics LIS344 ALH or Kionix KXM52. It should be highlighted that the accelerometer axes or sensing axes x, y and z are not necessarily coincident with the medio-lateral (ML), vertical (VT), and anterior-posterior (AP) body axes of the subject 100 since the accelerometer may be applied to the trunk of the subject in an arbitrary orientation and not perfectly defined position.

The accelerometer data indicative of a trunk motion of the subject is provided to the processing unit 10 for determining an ambulation motion of the subject 100. The processing apparatus 10 is configured to perform the steps shown in the flow chart of Fig. 2 as described further below.

In the embodiment shown in Fig. 1, the system 1 can further comprise an interface 6 and a memory 7. The interface 6 can be a wired or wireless interface. Advantageously, the system 1 can be a battery-powered device and the interface 6 is a wireless interface such that no cables are required. For example, the interface 6 can provide the accelerometer data acquired by the accelerometer 5 and/or a classification result of the processing unit 10 to an external entity.

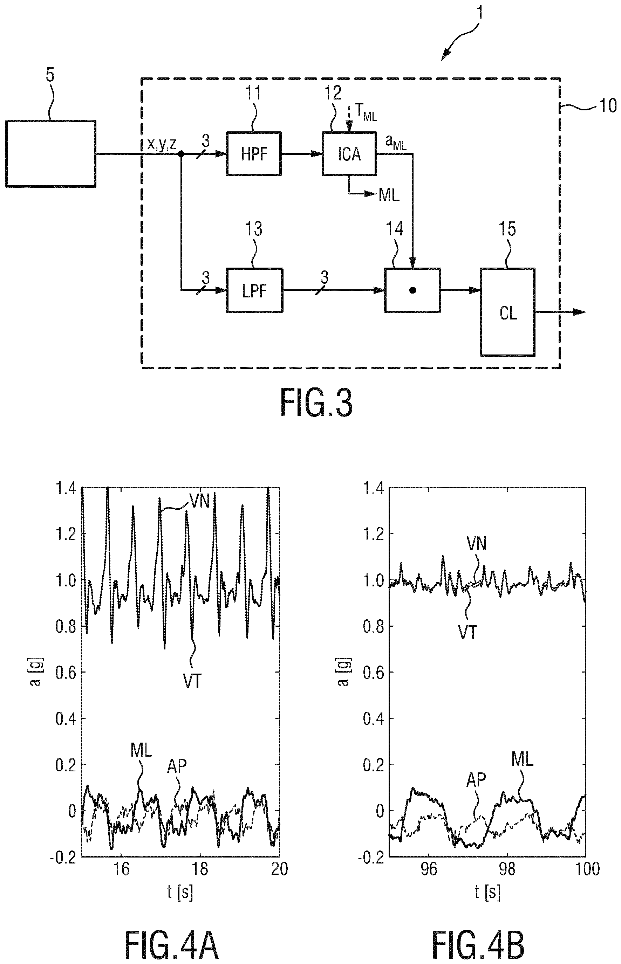

The memory 7 can store the accelerometer data indicative of a trunk motion of the subject 100 that has been acquired by the accelerometer 5. Alternatively or in addition, the memory 7 can be a non-transitory storage medium containing instructions for execution by the processing apparatus 10, wherein the instructions cause the processing apparatus 10 to perform the steps of the method disclosed herein, for example as described with reference to the flow chart shown in Fig. 2 and/or the block diagrams of Fig. 3, 9 or 10. For the case that the memory 7 stores the accelerometer data, the accelerometer data can, for example, acquired over a period of time, such that no connection to an external entity such as a smartphone or patient monitor is required. The accelerometer data can be downloaded after a desired measurement period. Thereby, the power consumption can be further reduced since no communication, in particular no wireless communication, is required.

In an alternative embodiment, the accelerometer 5 and the processing apparatus 10 are not implemented in one device. For example, a minimum configuration of accelerometer 5 and interface 6 can be implemented as a device that is worn by the subject 100. Accelerometer data can thus be transmitted via the interface 6 to the processing apparatus 10 at a remote location. For example, the processing apparatus 10 can be implemented as a part of a patient monitor or of a healthcare infrastructure such as a hospital IT system. The processing apparatus 10 or method can also be implemented as a cloud-based solution. In a further embodiment, a smartphone or a similar device can serves as the processing apparatus 10 and is configured to perform the steps for determining an ambulation motion of the subject 100.

Fig. 2 shows a flow chart of a method 20 comprising the steps performed by the processing apparatus 10 according to an aspect of the present invention. In a first step S21 accelerometer data indicative of a trunk motion of the subject can be obtained, i.e., received or retrieved. The accelerometer data can be a series of vectors, wherein each vector comprises the $x$-, $y$- and $z$-components of a tri-axial accelerometer. In a second step S22A, S22B at least one of a plurality of orthogonal motion components can be extracted from high-pass filtered accelerometer data. Thus, there is a first sub-step S22A of high-pass filtering the accelerometer data and a second sub-step S22B of extracting the at least one of a plurality of orthogonal motion components from the high-pass filtered accelerometer data. The second sub-step may comprise extracting a plurality of orthogonal motions components from the high-pas filtered accelerometer data. The use of a high-pass filter leads to accelerometer data which is free from an average gravity vector or gravity component. Thereby, the extraction of the at least one motion components is not biased based on a previous determination of a gravity component. In a third step S23A, S23B, a gravity component can be determined from low-pass filtered accelerometer data. Thus, there is a first sub-step S23A, S23B of low-pass filtering the accelerometer data and a second sub-step S23B of determining the gravity component from the low-pass filtered data. The second step S22A, S22B and the third step S23A, S23B can optionally be performed in series, in parallel or in different sequence. In a fourth step S24, a direction of at least one of said orthogonal motion components can be compared with a direction of the gravity component. Based thereon, in a fifth step S25, a motion of the subject can be classified as ambulation if said motion component is orthogonal or parallel to said gravity component. More than one motion component can be compared with the gravity component and used in the classification.

A further advantage of exploiting orthogonal motion component during ambulation can be that an orientation of the accelerometer 5 with respect to the trunk of the subject 100 can be determined. For example, an orientation of one or more motion components can be determined with respect to the sensing axes of the accelerometer. Based thereon, the accelerometer data can be calibrated, for example by applying a rotation matrix to obtain calibrated accelerometer data. Such calibrated accelerometer data can provide valuable information for example for reliably detecting a posture of the subject such as determining whether the subject lies on the left or right side when lying in bed.

Fig. 3 shows a schematic diagram of a second embodiment of a system 1 according to an aspect of the present invention. The system 1 comprises an accelerometer 5 for acquisition of accelerometer data indicative of a trunk motion the subject 100 and a processing apparatus 10 for determining an ambulation motion of the subject.

The processing apparatus 10 receives the accelerometer data as an input. The accelerometer data comprises three separate components, i.e., x, y and z components acquired by the three sensing axis of a tri-axial accelerometer 5. The three separate axes of the accelerometer data are first provided to a high-pass filter (HPF) 11. In the non-limiting embodiment of Fig. 3, the three high-pass filtered components of the accelerometer data are provided to an independent component analysis (ICA) unit 12 for extracting at least one of the orthogonal motion components based on independent component analysis as a source separation technique. The ICA unit 12 can provide at least one of the orthogonal motion components as an output. In the shown example, the ICA unit 12 is adapted to provide a medio-lateral axis $a_{ML}$, indicative of a direction of the medio-lateral motion component at its output. The ICA unit 12 can optionally be further configured to provide medio-lateral acceleration data ML in direction of the medio-lateral axis, also referred to as ML signal, as a further output. The ICA unit 12 can be adapted to extract the signal component with the lowest fundamental frequency by means of a specification of a time lag T provided at an input. In the shown example, a time lag of the ML motion component $T_{ML}$ of e.g. 200ms may be set. The ICA unit 12 can determine the ML motion component by maximizing a normalized autocorrelation at the set time lag. This extracts the ML signal or sideways signal and further provides the ML axis as an identification of a sideways axis.

Parallel to the high-pass filtering, the (raw) accelerometer data is provided to a low-pass filter (LPF) 13 for determining a gravity component. Hence, the gravity component may simply be determined by low-pass filtering or smoothing the accelerometer data with a LPF having a sufficiently low cut-off frequency, or equivalently by averaging the accelerometer data over a sufficiently long period of time such as some seconds. This provides the direction of the gravity component.

In the embodiment shown in Fig. 3, the direction of the ML component is compared with said direction of the gravity component or gravity vector by computing the dot-product by means of a comparison unit 14. The dot-product of the identified ML axis with the direction of the gravity vector should be close to zero for walking because the ML axis should be perpendicular to the sagittal plane. Hence, the output of the comparison unit 14 can be provided as an input to the classifier (CL) 15. Thus, the motion of the subject can be classified as ambulation if the ML component is orthogonal to the gravity component.

In the given example, the motion component can deemed to be orthogonal to the gravity component if an absolute value of a dot product of the motion component and the gravity component is below a predetermined threshold.

Figs. 4A and 4B show diagrams illustrating accelerometer data acquired by a tri-axial accelerometer applied to a trunk of as well as the vector norm (VN) for a fast walking subject and a slow walking subject, respectively. The horizontal axis denotes the time t in seconds [s] whereas the vertical axis denotes the measured acceleration in [g]. For illustration, the accelerometer axes in the given example are aligned with the body axes of the subject, wherein x-axis ~ ML, y-axis ~ VT, and z-axis ~ AP component. Hence, the traces shown in Fig. 4A and 4B correspond the three orthogonal motion components (1) medio-lateral (ML), (2) anterior-posterior (AP), and (3) vertical (VT). The dotted trace denotes the vector norm (VN) computed as:

$$VN(t) = \sqrt{ML^2(t) + AP^2(t) + VT^2(t)}.$$

As can be seen, in Figure 4A and 4B, the vector norm is nearly equal to the VT component. When decomposing the accelerometer data into a gravity component (=1g) and the inertial components, it is known that inertial components orthogonal to the gravity vector are attenuated in the vector norm, whereas inertial components in the same direction as the gravity vector are enhanced in the vector norm. Furthermore, it can be seen from Fig. 4A and 4B that the large ML components that can originate from rotations of the sensor are not visible anymore in the vector norm. This can be attributed to the gravity vector component being distributed over all orthogonal components in such a way that the vector norm stays the same, being the gravity acceleration component plus inertial accelerations measured mainly in the direction perpendicular to the gravity vector.

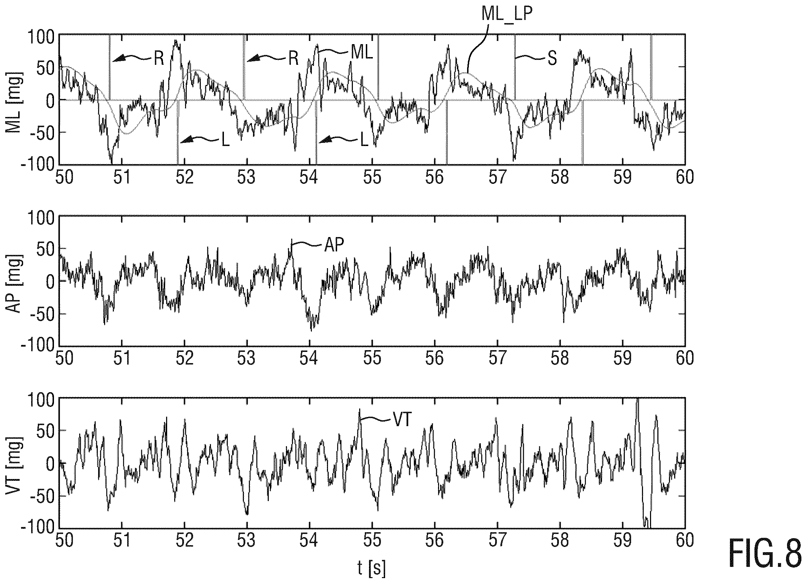

It should be noted that step-detection based on the vector norm are often used in conventional approaches. An advantage of this approach is that step-detection based on the vector norm is rotation-independent. Step detection based on the vector norm works well for fast walking. As can be seen in Fig. 4A, the vertical (VT) component, which is nearly equal to the vector norm, is strong for fast walking and individual steps can be clearly identified. In Fig. 4B, 8 steps can be identified in the trace of 5 seconds. These 8 steps are equal to 4 strides, which is visible in the ML component.

On the other hand, it has been found that the resulting VN signal is rather small for slow walking as shown in Fig. 4B. Moreover, the individual steps cannot be clearly identified. In the solution disclosed herein it is therefore in particular suggested to use the ML and AP components for step-detection. It has been found that this can lead to a more robust step-detection for slow walking or a slow ambulation motion of the subject. As can be seen in Fig. 4B, the medio-lateral (left-to-right) and anterior-posterior (front-to-back) motion components can be clearly identified. In Fig. 4B, the ML component is strongest and roughly 2 ½ strides corresponding to 5 steps can be identified in the trace of 5 seconds.

The ML component has a fundamental frequency that is typically half the fundamental frequency of the VT and AP component, because of the lateral / sideways movement of the trunk for each subsequent left/right step. Furthermore, the AP component has the same fundamental frequency as the VT component, but has a different temporal structure. The VT component shows with each step a number of 'spikes' where the time just before the largest spike reflects the time of heel-impact and the 'valley' just after the largest spike reflects the toe-off. The AP component on the other hand has a ramp-like structure. The ramp resets to a minimum value and rises again during toe-off. It has been found that such general temporal characteristics can be seen in both slow and fast walking, although the morphology and amplitudes may differ. For example, the VT component is much less dominant for slow walking.

It is thus suggested to extract one or more different orthogonal motion components, here the three components VT, AP and ML, from high-pass filtered accelerometer data by using source extraction techniques. Source extraction techniques can be based on the concept of linear predictability. This approach assumes that the source signals have some temporal structure. For example the sources have different (normalized) autocorrelation functions or equivalently have different (normalized) spectral shapes. Intuitively speaking, the source signals have less complexity than the mixed sensor signals. In other words, the degree of temporal predictability of any source signal is higher than (or equal to) that of any mixture. For example, waveforms of a mixture of two sine waves with different frequencies are more complex or less predictable than either of the original sine waves. Exemplary memory efficient and cost effective iterative structures for the source separation are described in the aforementioned review paper by Choi et al.

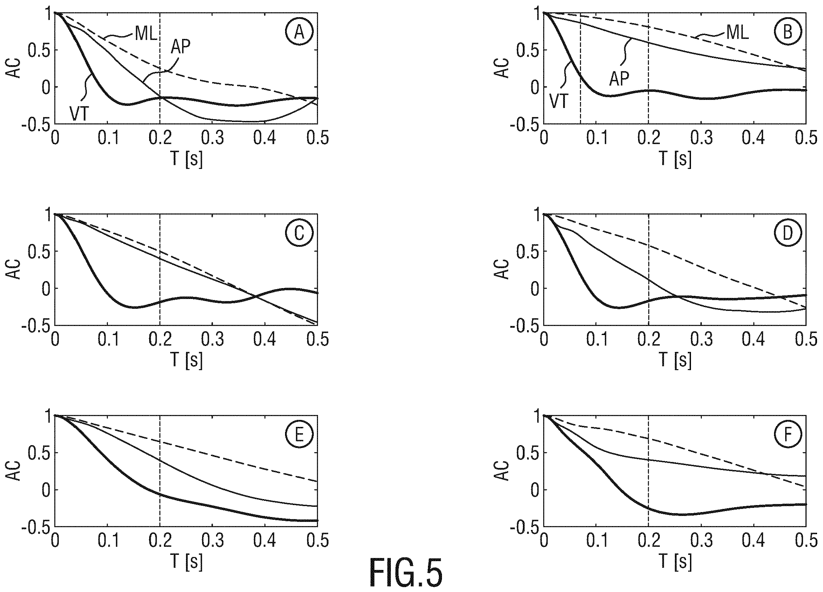

Fig. 5 shows exemplary diagrams of an autocorrelation for three orthogonal motion components for different types of ambulation: A) walking fast, B) walking slow, C) walking asymmetric, D) walking irregular, E) stairs up, and F) stairs down. The horizontal axis denotes the time lag T in seconds [s], whereas the vertical axis denotes a normalized (unity at lag 0) autocorrelation coefficient (AC).

As can be seen from Fig. 5 the ML component shows the highest autocorrelation, for example at a time lag of 200 ms, as indicated by the vertical dashed line. The ML component can thus be extracted by setting the source separation algorithm to extract, i.e. to separate, the ML component as the desired source from the other sources by maximizing the auto-correlation coefficient at a predetermined time lag, here 200 ms. The extracted ML component can then be used for detection of steps in slow walking because the other orthogonal components (VT and AP) are very small when people are walking slowly, as illustrated in Fig. 4B.

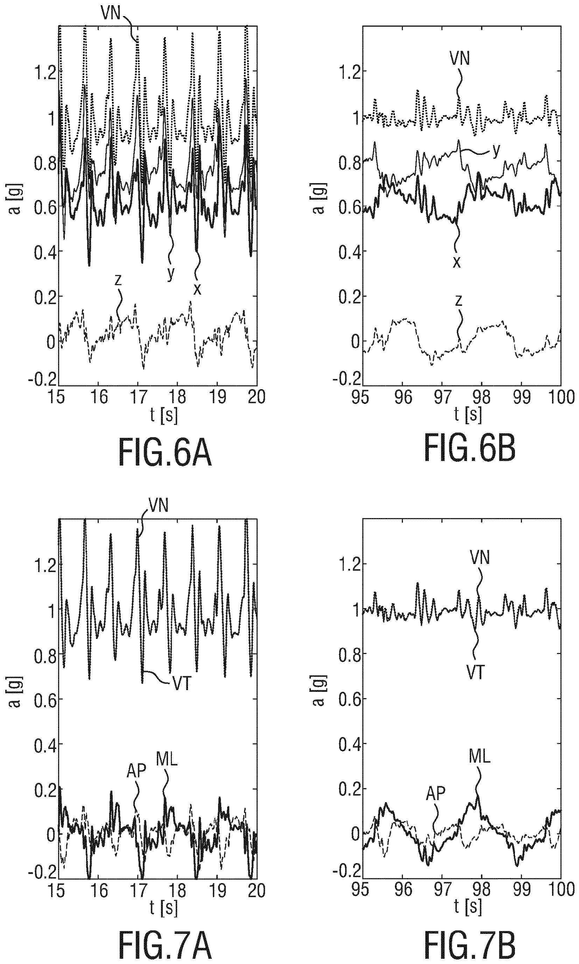

Figs. 6A and 6B show diagrams illustrating accelerometer data acquired by a tri-axial accelerometer applied to a trunk of a subject as well as the vector norm for fast walking and slow walking, respectively. The horizontal axis again denotes the time t in seconds [s] whereas the vertical axis denotes the acceleration in [g]. In contrast to Figs. 4A and 4B, the accelerometer axes in Figs. 6A and 6B are not aligned with the body axes of the subject. For a fair comparison, the same data is used as in Figs. 4A and 4B but with a rotated coordinate system. This corresponds to the situation where the accelerometer is applied at in an arbitrarily rotated orientation, i.e., not aligned with the body axes of the subject.

By comparing Figs. 6A and 6B to Figs. 4A and 4B it can be seen that the vector norm is the same for both cases.

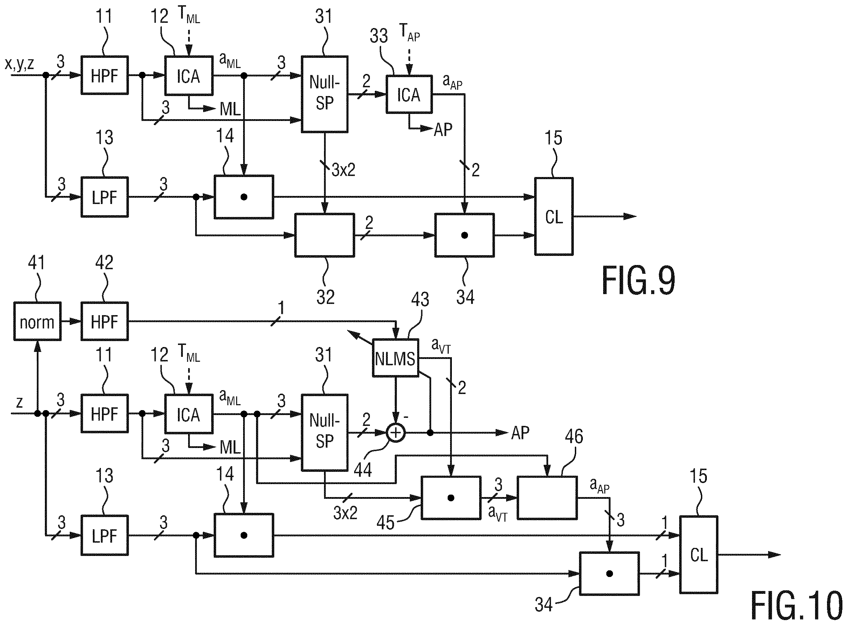

The accelerometer data shown in Figs. 6A and 6B, acquired with an arbitrarily oriented accelerometer, can be provided as an input to the processing unit 10, as for example described with reference to Fig. 3 or later with reference to Figs. 9 or 10. In particular the medio-lateral motion component can be extracted based on the high-pass filtered acquired x, y and z accelerometer data by choosing an appropriate auto-correlation time lag as described above.

Based on the first extracted component, such as the ML component, a plane perpendicular to said first component can be determined, being the sagittal plane. In the given example, the sagittal plane comprises a mixture of the remaining orthogonal components, here VT and AP. These components can be extracted using a similar source-separation algorithm, but with a two-vector input and using a different time-lag. From the auto-correlation functions in Fig. 5, it follows that for the separation of the VT and AP components the use of a smaller time-lag for the extraction is appropriate. Hence, a deflation-technique can be used. The extraction can start with a 3 source-mixture. A first source is extracted, e.g. the ML component. Based thereon, the dimension can be reduced to a mixture of two sources and a similar source-separation technique can be applied to extract the second source, e.g. the AP component. The third source (orthogonal to the other two sources) is remaining. The deflation-technique can thus be seen as a step-wise approach of finding the orthogonal components. In the given example, there are two steps. The first step is to extract the ML component from the tri-axial accelerometer data. After this identification of the ML component, the subspace orthogonal to the ML component (which ideally contains only the AP and VT component) is taken. The second step is then to extract from this two-dimensional subspace the AP component. The VT component is the remainder. The result of applying this source separation to extract the ML, AP and VT components form the arbitrarily oriented accelerometer data of Figs. 6A and 6B is shown in Fig. 7A and 7B.

When comparing Figs. 7A and 7B to Figs. 4A and 4B, wherein the accelerometer axes are carefully aligned with the ML, AP and VT axes, it can be seen that the sources are extracted with good correspondence by the deflation source-separation technique exploiting the temporal structure. In the non-limiting example shown herein, a 200 ms time lag is used for the extraction of the ML component, whereas a 75 ms time lag is used for the extraction of the AP component.

It shall be understood that a sign ambiguity in the source extraction can be recovered, for example, by exploiting an indication for use and/or exploiting additional a priori information. For example, in case of combined monitoring of ECG and ambulation motion it is required that the device is worn on the chest. Furthermore, a priori information about the orientation of the accelerometer with respect to an adhesive side of the patch can be exploited. In a further example, as exemplarily shown in Fig. 1, the accelerometer is typically only slightly rotated around an axes in the coronal plane and the sign for the AP component can be recovered based thereon. Furthermore, the VT component has to correlate positively with the (DC-removed) vector norm (VN). If the correct signs for the AP and the VT component are known and the coordinate system of the tri-axial accelerometer sensor is known, also the sign of the ML component can be recovered. It is thus possible to determine if a particular zero-crossing of the ML component belongs to either a left or right step).

In order to determine an ambulation motion of the subject, a direction at least one of said extracted motion components in the high-pass filtered raw accelerometer data can be compared with a direction of the gravity component computed by the low-pass filtered raw accelerometer data, e.g. as an averaged VN. If the direction of at least one the components is orthogonal or parallel to the gravity-vector, the accelerometer observation can be classified as being ambulation, whereas if the direction of at least one of the components is neither substantially orthogonal nor parallel to the gravity component, the accelerometer observation can be classified as non-ambulation.

Optionally, after having classified the accelerometer observation as being ambulation, one or more of the extracted orthogonal components can be used to identify individual steps during ambulation. For example, the rising and falling zero-crossing of the ML components can be used to compute the step-count or valleys in the AP component, preferably a low-pass filtered or integrated AP component, can be identified to pinpoint a heel-strike moment, where the velocity drops due to the heel-strike. In an embodiment, the integration in the AP component can be used to derive the velocity of the AP acceleration component. Furthermore, other gait-features may be computed from one or more of the three orthogonal components. For example a step-length can be determined when exploiting the AP component or via an inverse pendulum model exploiting the VT component.

Fig. 8 shows exemplary diagrams of extracted orthogonal motion components for a slow walking subject walking at a speed of 0.5 m/s. The horizontal axis denotes the time t in seconds [s], whereas the vertical axis denotes the acceleration in [mg] for the respective orthogonal motion component. The top graph refers to the medio-lateral (ML) component, the middle graph to the anterior-posterior (AP) component, and the bottom graph to the vertical (VT) component.

The top graph in Fig. 8 further shows a trace of the medio-lateral component after smoothing or low-pass filtering, denoted by ML_LP. For identifying individual steps the zero-crossings of ML LP can be evaluated. In the alternative or in addition valleys of for example the AP component, preferably after smoothing or first-order integration, can be evaluated to identify individual steps. Optionally, individual left and right steps can be identified based on rising and falling zero-crossings, advantageously after smoothing based on ML LP. Exemplary left and right steps are indicated by L and R in the top graph of Fig. 8.

Fig. 9 shows a schematic diagram of a further embodiment of the device 10 according to an aspect of the present invention. Since the embodiment is based on the device 10 as described with reference to Fig. 3, only differences and/or additional aspects will be described in the following.

After having identified a first motion component, here the ML component using a first independent component analysis (ICA) unit 12, a dimensionality reduction stage or deflation can be used to find one or more further motion components indicative of an ambulation motion of the subject (here VT and AP).

Said deflation can comprise a null-space block 31 adapted to receive the axis of the first motion component, here $a_{ML}$, and the high-pass filtered accelerometer data as inputs. The null-space block 31 can comprise two outputs. The first output can be a two-dimensional output that is the sub-space signal, which does not include the first motion component anymore. In other words, it contains the sub-space signal corresponding to the null-space of said first motion component. In other words, the first motion component can be removed by means of projection/deflation. This leaves a 1-dimensional null-space in the 3-dimensional space. The null-space block 31 can perform a dimensionality reduction and removes the null-space (which in the given example is in the ML direction), resulting in a 2-dimensional signal space also referred to as sub-space. Hence, based on the high-pass filtered accelerometer data and said first motion component, a two-dimensional sub-space orthogonal to said first motion component can be determined. Based thereon, similar to the determination of the first motion component by the first ICA unit 12, a second motion component can be extracted based on said sub-space. Different approaches will be described with reference to Fig. 9 and Fig. 10.

In the embodiment shown in Fig. 9, the second motion component, here the AP component is extracted by a source separation technique, for example by a second independent component analysis (ICA) unit 33. Since an autocorrelation function of the VT component is less wide compared to the AP component, as shown in Fig. 5, a similar ICA as described with reference to Fig. 3 can be applied, however now based on the two-dimensional sub-space and specifying a different time lag. In the given example, the time lag $T_{AP}$ is tuned to 75 ms to extract the AP signal. The second ICA unit 33 thus provides the AP axis $a_{AP}$ and an accelerometer data along the anterior-posterior axis AP as its outputs. The AP axis can be provided as a first input to a second comparison unit 34. It shall be understood that the comparison unit may determine, for example, a dot-product, vector-product or in-product, more generally speaking a feature value that can be used in the classifier for classifying a motion of the subject as ambulation, for example, by comparing the feature value with one or more threshold values.

The second output of the null-space block can be a 3x2 matrix that defines the sub-space by two three-dimensional vectors. This 3x2 matrix can be used in a subsequent processing block 32 to compute another sub-space signal. As explained above, the first motion component which has already been extracted or determined can thus be removed by means of projection or deflation. The processing block 32 may receive the 3x2 matrix as a first input and the low-pass filtered accelerometer data provided by the low-pass filter 13 as a second input and compute a vector product based thereon. Hence, the projection of the accelerometer data on the sub-space can be done for both the HPF signal and the LPF signal. The output of processing block 32 is provided as a second input to a second comparison unit 34.

In the embodiment shown in Fig. 9, the direction of the AP component can again be compared with the direction of the gravity component by computing the dot-product by means of a comparison unit 34. The output of the processing block 32 can be seen as projection of the gravity component in the transverse plane. The output of the second comparison unit 34 can be used as a second input to the classifier unit 15 for classifying a motion of the subject as ambulation as explained above with reference to Fig. 3.

Fig. 10 shows a schematic diagram of another embodiment of the device according to an aspect of the present invention. Since the embodiment is based on the device 10 as described with reference to Fig. 3 and/or Fig. 9, only differences and/or additional aspects will be described in the following. This embodiment describes an alternative approach for extracting a second motion component, here the AP and/or VT component.

For the embodiment shown in Fig. 10, the extraction of the medio-lateral component can be the same as in the previous embodiments. Furthermore, the processing unit 10 can be configured to extract a first motion component based on a source separation technique; determine, based on the high-pass filtered accelerometer data and said first motion component, a two-dimensional sub-space orthogonal to said first motion component, similar to the embodiment described with reference to Fig. 9. However, as an alternative to a second ICA stage, the processing unit 10 can be configured to extract said second motion component based on said sub-space and a high-pass filtered norm or vector norm of the accelerometer data. In block 41, the vector norm of the accelerometer data is computed and provided to a high-pass filter (HPF) 42. The high-pass filtered norm of the accelerometer data can be used as a reference signal for the extraction of the vertical component from the accelerometer data.

Based thereon, an NLMS (normalized least mean squares) technique can be used in block 43 to find a correlation between the high-pass filtered norm with the high-pass filtered accelerometer data or the output of the null-space block 31 indicative of the high-pass filtered accelerometer data without the ML component. The reason why the norm of the accelerometer data correlates with the vertical component of the accelerometer data is because the norm (without high-pass filter) contains a large gravity component. All accelerations orthogonal to the gravity vector are suppressed in the norm because of this. The VT component can be found by maximizing the correlation. The axis of the VT component $a_{VT}$ can be provided as an output of the NLMS block 43. Based on the subtraction operation in block 44, the AP accelerometer data can be obtained.

In other words, the NLMS block shown in Fig. 10 can receive a reference signal, such as the high-pass filtered vector norm, and also the subspace provided by the null-space block which ideally comprises the high-pass filtered AP and VT acceleration data. Based thereon, the NLMS can perform a correlation such that the VT direction can be identified in this subspace. A residual signal provided by the NLMS block 43 can contain, after subtraction of the VT acceleration, the AP acceleration data, as shown in Fig. 10.

After extracting (determining) the VT component in the two-dimensional subspace, e.g. by using the NLMS, the VT component in a three-dimensional subspace can be calculated by the vector-matrix product of the VT direction in a two-dimensional subspace with the 3x2 null-space matrix in block 31. Subsequently, the AP direction can be determined, by computing the cross-product between the VT and the ML component. More generally a third orthogonal motion component can be determined based on the cross product of a first orthogonal motion component and a second orthogonal motion component.

As an alternative, in block 45 a vector-matrix product of the 3x2 matrix provided by the null-space block 31 and the VT axis $a_{VT}$ can be calculated. In shall be understood that the output of block 43 can represents the VT direction $a_{VT}$ in the two-dimensional subspace. The output of the vector-matrix product of block 45 can represent the VT direction $a_{VT}$ in three-dimensional space. Based thereon the cross-product with the axis of the ML component $a_{ML}$ can be computed, in block 46. The output of this block gives the axis of the AP component $a_{AP}$, which can then be provided to a second comparison unit 34 for comparison with a direction of the gravity component. In the shown embodiment, the gravity component can again be provided as the output of low-pass filter (LPF) 13.

The classification step of the embodiment of Fig. 10 can again be similar to the previous embodiment. The classifier 15 may check if the directions of the determined ML and/or AP components are orthogonal to the direction of the gravity component to classify a motion of the subject as ambulation.

An advantage of the solution shown in Fig. 10 over the solution in Fig. 9 can be that the robustness can be further improved. This embodiment thus takes into consideration that the differences in the normalized auto-correlations between the VT vs. AP components are less pronounces than the differences in the normalized auto-correlation between the ML vs. AP or VT components, as illustrated in Fig. 5. Hence, the NLMS (and exploiting the vector-norm VN) can be used as an alternative or in addition to the two-stage ICA approach shown in Fig. 9.

In conclusion the solution proposed herein, in particular extracting an ML component from high-pass filtered accelerometer data (only) and comparing the direction of the ML component with a direction of the gravity component, and classifying a motion of the subject as ambulation based thereon, provide a possibility to reliably determine an ambulation motion of the subject in particular for slow moving subjects. This can be advantageous in obtaining context information for vital signs measurements for sick and/or elderly persons.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A processing apparatus (10) for determining an ambulation motion of a subject (100), the processing apparatus configured to perform the steps of:

obtaining accelerometer data indicative of a trunk motion of the subject (S21);

performing a high pass filter on the accelerometer data to obtain high-pass filtered accelerometer data;

extracting at least one of a plurality of orthogonal motion components from the high-pass filtered accelerometer data (S22B) using a source-separation technique;

performing a low-pass filter on the accelerometer data to obtain low-pass filtered accelerometer data;

determining a gravity component from the low-pass filtered accelerometer data (S23B);

comparing a direction of at least one of said orthogonal motion components with a direction of the gravity component (S24); and

classifying a motion of the subject as ambulation if said motion component is orthogonal or parallel to said gravity component (S25).

2. The processing apparatus as claimed in claim 1, configured to extract a medio-lateral (ML) and/or an anterior-posterior (AP) motion component from said high-pass filtered accelerometer data.

3. The processing apparatus as claimed in claim 1, wherein the source separation technique is based on one or more of: linear predictability, blind source separation (BSS) and/or independent component analysis (ICA).

4. The processing apparatus as claimed in claim 1, wherein said source-separation technique is adapted to separate at least one of said orthogonal components based on their temporal structure, in particular by maximizing an auto-correlation at a predetermined time lag.

5. The processing apparatus as claimed in claim 4, wherein said source separation technique is adapted to maximize an auto-correlation at a predetermined time lag, wherein said time lag is between 100 ms and 500 ms, preferably between 150 ms and 400 ms, preferably between 200 ms and 350 ms.

6. The processing apparatus as claimed in claim 4, configured to extract a first orthogonal motion component based on a source-separation technique, wherein said source-separation technique is adapted to maximize an auto-correlation at a first predetermined time lag; and further configured to extract a second orthogonal motion component based on a source-separation technique, wherein said source-separation technique is adapted to maximize an auto-correlation at a second predetermined time lag.

7. The processing apparatus as claimed in claim 1, wherein the step of extracting at least one of a plurality of orthogonal motion components comprises:

extracting a first motion component based on a source separation technique;

determining, based on the high-pass filtered accelerometer data and said first motion component, a two-dimensional sub-space orthogonal to said first motion component; and

extracting a second motion component based on said sub-space.

8. The processing apparatus as claimed in claim 7, configured to extract said second motion component based on said sub-space and a high-pass filtered norm of the accelerometer data.

9. The processing apparatus as claimed in claim 1, wherein in the step of classifying a motion of the subject as ambulation the motion component is deemed to be orthogonal to the gravity component if an absolute value of a dot product of the motion component and the gravity component is below a predetermined threshold.

10. The processing apparatus as claimed in claim 1, further configured to perform the step of calibrating the accelerometer data based on the at least one extracted orthogonal motion component.

11. The processing apparatus as claimed in claim 1, further configured to perform the step of identifying individual steps and/or identifying a gait parameter during ambulation based on the at least one the extracted orthogonal motion component.

12. The processing apparatus as claimed in claim 11, configured to identify individual left and right steps based on rising and falling zero-crossings of a medio-lateral motion component and/or based on valleys in a vertical or anterior-posterior motion component.

13. System (1) for determining an ambulation motion of a subject, the system comprising:

an accelerometer (5) for acquisition of accelerometer data indicative of a trunk motion the subject; and

the processing apparatus (10) for determining an ambulation motion of the subject as claimed in claim 1.

14. A method (20) performed by a processing apparatus (10) for determining an ambulation motion of a subject, the method comprising the steps of:

obtaining accelerometer data indicative of a trunk motion of the subject (S21),
performing (S22A) a high pass filter on the accelerometer data to obtain high-pass filtered accelerometer data;
extracting at least one of a plurality of orthogonal motion components from the high-pass filtered accelerometer data (S22B) using a source separation technique;
performing (S22B) a low-pass filter on the accelerometer data to obtain low-pass filtered accelerometer data;
determining a gravity component from the low-pass filtered accelerometer data (S23B);
comparing a direction of at least one of said orthogonal motion components with a direction of the gravity component (S24); and
classifying a motion of the subject as ambulation if said motion component is orthogonal or parallel to said gravity component (S25).

15. Computer program comprising program code means for causing a computer to carry out the steps of the method as claimed in claim 14 when said computer program is carried out on the computer.

**Patentansprüche**

1. Verarbeitungseinrichtung (10) zur Bestimmung einer Gehbewegung eines Patienten (100), wobei die Verarbeitungseinrichtung konfiguriert ist, um die folgenden Schritte durchzuführen:

Erhalten von Beschleunigungsmesserdaten, die auf eine Rumpfbewegung des Patienten (S21) hinweisend sind;
Durchführen eines Hochpassfilters auf den Beschleunigungsmesserdaten, um hochpassgefilterte Beschleunigungsmesserdaten zu erhalten;
Extrahieren mindestens einer von einer Vielzahl von orthogonalen Bewegungskomponenten aus den hochpassgefilterten Beschleunigungsmesserdaten (S22B) unter Verwendung einer Quellentrenntechnik;
Durchführen eines Tiefpassfilters auf den Beschleunigungsmesserdaten, um tiefpassgefilterte Beschleunigungsmesserdaten zu erhalten;
Bestimmen einer Schwerkraftkomponente von den tiefpassgefilterten Beschleunigungsmesserdaten (S23B);
Vergleichen einer Richtung von mindestens einer der orthogonalen Bewegungskomponenten mit einer Richtung der Schwerkraftkomponente (S24); und
Klassifizieren einer Bewegung des Patienten als Gehen, falls die Bewegungskomponente orthogonal oder parallel zur Schwerkraftkomponente (S25) ist.

2. Verarbeitungseinrichtung nach Anspruch 1, konfiguriert, um eine mediolaterale (ML) und/oder eine anterior-posteriore (AP) Bewegungskomponente aus den hochpassgefilterten Beschleunigungsmesserdaten zu extrahieren.

3. Verarbeitungseinrichtung nach Anspruch 1, wobei die Quellentrenntechnik auf einem oder mehreren von Folgendem basiert: linearer Vorhersagbarkeit, blinder Quellentrennung (Blind Source Separation - BSS) und/oder Unabhängigkeitsanalyse (Independent Component Analysis - ICA).

4. Verarbeitungseinrichtung nach Anspruch 1, wobei die Quellentrenntechnik angepasst ist, um mindestens eine der orthogonalen Komponenten basierend auf ihrer zeitlichen Struktur zu trennen, insbesondere durch Maximieren einer Auto-Korrelation bei einer vorbestimmten Zeitverzögerung.

5. Verarbeitungseinrichtung nach Anspruch 4, wobei die Quellentrenntechnik angepasst ist, um eine Auto-Korrelation bei einer vorbestimmten Zeitverzögerung zu maximieren, wobei die Zeitverzögerung zwischen 100 ms und 500 ms liegt, vorzugsweise zwischen 150 ms und 400 ms, vorzugsweise zwischen 200 ms und 350 ms.

6. Verarbeitungseinrichtung nach Anspruch 4, konfiguriert, um eine erste orthogonale Bewegungskomponente basierend auf einer Quellentrenntechnik zu extrahieren, wobei die Quellentrenntechnik angepasst ist, um eine Auto-Korrelation bei einer ersten vorbestimmten Zeitverzögerung zu maximieren; und weiter konfiguriert, um eine zweite orthogonale Bewegungskomponente basierend auf einer Quellentrenntechnik zu extrahieren, wobei die Quellentrenntechnik angepasst ist, um eine Auto-Korrelation bei einer zweiten vorbestimmten Zeitverzögerung zu maximieren.

7. Verarbeitungseinrichtung nach Anspruch 1, wobei der Schritt des Extrahierens mindestens einer von einer Vielzahl von orthogonalen Bewegungskomponenten umfasst:

Extrahieren einer ersten Bewegungskomponente basierend auf einer Quellentrenntechnik;
Bestimmen, basierend auf den hochpassgefilterten Beschleunigungsmesserdaten und der ersten Bewegungskomponente, eines zweidimensionalen Unterraumes, der orthogonal zur ersten Bewegungskomponente ist; und
Extrahieren einer zweiten Bewegungskomponente basierend auf dem Unterraum.

**8.** Verarbeitungseinrichtung nach Anspruch 7, konfiguriert, um die zweite Bewegungskomponente basierend auf dem Unterraum und einer hochpassgefilterten Norm der Beschleunigungsmesserdaten zu extrahieren.

**9.** Verarbeitungseinrichtung nach Anspruch 1, wobei im Schritt des Klassifizierens einer Bewegung des Patienten als Gehen die Bewegungskomponente als orthogonal zur Schwerkraftkomponente angesehen wird, falls ein absoluter Wert eines Skalarprodukts der Bewegungskomponente und der Schwerkraftkomponente unterhalb einer vorbestimmten Schwelle liegt.

**10.** Verarbeitungseinrichtung nach Anspruch 1, weiter konfiguriert, um den Schritt des Kalibrierens der Beschleunigungsmesserdaten basierend auf der mindestens einen extrahierten orthogonalen Bewegungskomponente durchzuführen.

**11.** Verarbeitungseinrichtung nach Anspruch 1, weiter konfiguriert, um den Schritt des Identifizierens individueller Schritte und/oder Identifizierens eines Gangparameters während des Gehens basierend auf der mindestens einen der extrahierten orthogonalen Bewegungskomponente durchzuführen.

**12.** Verarbeitungseinrichtung nach Anspruch 11, konfiguriert, um individuelle Links- und Rechtsschritte basierend auf steigenden und fallenden Nulldurchgängen einer medio-lateralen Bewegungskomponente und/oder basierend auf Tälern in einer vertikalen oder anterior-posterioren Bewegungskomponente zu identifizieren.

**13.** System (1) zur Bestimmung einer Gehbewegung eines Patienten, wobei das System umfasst:

einen Beschleunigungsmesser (5) zum Erlangen von Beschleunigungsmesserdaten, die auf eine Rumpfbewegung des Patienten hinweisend sind; und
die Verarbeitungseinrichtung (10) zum Bestimmen einer Gehbewegung des Patienten nach Anspruch 1.

**14.** Verfahren (20), durchgeführt durch eine Verarbeitungseinrichtung (10) zum Bestimmen einer Gehbewegung eines Patienten, wobei das Verfahren die folgenden Schritte umfasst:

Erhalten von Beschleunigungsmesserdaten, die auf eine Rumpfbewegung des Patienten (S21) hinweisend sind,
Durchführen (S22A) eines Hochpassfilters auf den Beschleunigungsmesserdaten, um hochpassgefilterte Beschleunigungsmesserdaten zu erhalten;
Extrahieren mindestens einer von einer Vielzahl von orthogonalen Bewegungskomponenten aus den hochpassgefilterten Beschleunigungsmesserdaten (S22B) unter Verwendung einer Quellentrenntechnik;
Durchführen (S22B) eines Tiefpassfilters auf den Beschleunigungsmesserdaten, um tiefpassgefilterte Beschleunigungsmesserdaten zu erhalten;
Bestimmen einer Schwerkraftkomponente aus den tiefpassgefilterten Beschleunigungsmesserdaten (S23B);
Vergleichen einer Richtung von mindestens einer der orthogonalen Bewegungskomponenten mit einer Richtung der Schwerkraftkomponente (S24); und
Klassifizieren einer Bewegung des Patienten als Gehen, falls die Bewegungskomponente orthogonal oder parallel zur Schwerkraftkomponente (S25) ist.

**15.** Computerprogramm, umfassend Programmcodemittel, um zu bewirken, dass ein Computer die Schritte des Verfahrens nach Anspruch 14 ausführt, wenn das Computerprogramm auf dem Computer ausgeführt wird.

**Revendications**

**1.** Appareil de traitement (10) pour déterminer un mouvement ambulatoire d'un sujet (100), l'appareil de traitement étant configuré pour réaliser les étapes consistant à :

obtenir des données d'accéléromètre indiquant un mouvement de tronc du sujet (S21) ;
réaliser un filtrage passe-haut sur les données d'accéléromètre pour obtenir des données d'accéléromètre filtrées passe-haut ;
extraire au moins l'un parmi une pluralité de composantes de mouvement orthogonal à partir des données d'accéléromètre filtrées passe-haut (S22B) en utilisant une technique de séparation de source ;
réaliser un filtrage passe-bas sur les données d'accéléromètre pour obtenir des données d'accéléromètre filtrées passe-bas ;
déterminer une composante de gravité à partir des données d'accéléromètre filtrées passe-bas (S23B) ;
comparer une direction d'au moins l'une desdites composantes de mouvement orthogonal avec une direction de la composante de gravité (S24) ; et
classer un mouvement du sujet en tant qu'ambulation si ladite composante de mouvement est orthogonale ou parallèle à ladite composante de gravité (S25).

2. Appareil de traitement selon la revendication 1, configuré pour extraire une composante de mouvement médio-latérale (ML) et/ou antéro-postérieure (AP) à partir desdites données d'accéléromètre filtrées passe-haut.

3. Appareil de traitement selon la revendication 1, dans lequel la technique de séparation de source est basée sur un ou plus parmi : une prédictabilité linéaire, une séparation de source en aveugle (BSS) et/ou une analyse de composante indépendante (ICA).

4. Appareil de traitement selon la revendication 1, dans lequel ladite technique de séparation de source est adaptée pour séparer au moins l'une desdites composantes orthogonales sur la base de leur structure temporelle, en particulier en maximisant une auto-corrélation à un temps de latence prédéterminé.

5. Appareil de traitement selon la revendication 4, dans lequel ladite technique de séparation de source est adaptée pour maximiser une auto-corrélation à un temps de latence prédéterminé dans lequel ledit temps de latence se situe entre 100 ms et 500 ms, de préférence entre 150 ms et 400 ms, de préférence entre 200 ms et 350 ms.

6. Appareil de traitement selon la revendication 4, configuré pour extraire une première composante de mouvement orthogonal sur la base d'une technique de séparation de source, dans lequel ladite technique de séparation de source est adaptée pour maximiser une auto-corrélation à un premier temps de latence prédéterminé ; et configuré en outre pour extraire une deuxième composante de mouvement orthogonal sur la base d'une technique de séparation de source, dans lequel ladite technique de séparation de source est adaptée pour maximiser une auto-corrélation à un deuxième temps de latence prédéterminé.

7. Appareil de traitement selon la revendication 1, dans lequel l'étape d'extraction d'au moins l'une parmi une pluralité de composantes de mouvement orthogonal comprend :

une extraction d'une première composante de mouvement sur la base d'une technique de séparation de source ;
une détermination, sur la base des données d'accéléromètre filtrées passe-haut et de ladite première composante de mouvement, d'un sous-espace bidimensionnel orthogonal par rapport à ladite première composante de mouvement ; et

une extraction d'une deuxième composante de mouvement sur la base dudit sous-espace.

8. Appareil de traitement selon la revendication 7, configuré pour extraire ladite deuxième composante de mouvement sur la base dudit sous-espace et d'une norme filtrée passe-haut des données d'accéléromètre.

9. Appareil de traitement selon la revendication 1, dans lequel dans l'étape de classement d'un mouvement du sujet en tant qu'ambulation, la composante de mouvement est jugée orthogonale par rapport à la composante de gravité si une valeur absolue d'un produit scalaire de la composante de mouvement et de la composante de gravité se situe en-dessous d'un seuil prédéterminé.

10. Appareil de traitement selon la revendication 1, configuré en outre pour réaliser l'étape d'étalonnage des données d'accéléromètre sur la base de l'au moins une composante de mouvement orthogonal extraite.

11. Appareil de traitement selon la revendication 1, configuré en outre pour réaliser l'étape d'identification d'étapes individuelles et/ou d'identification d'un paramètre de marche pendant une ambulation sur la base de l'au moins une composante de mouvement orthogonal extraite.

12. Appareil de traitement selon la revendication 11, configuré pour identifier des étapes gauche et droite individuelles sur la base d'une augmentation et diminution des points de passage de zéro d'une composante de mouvement médio-latérale et/ou sur la base de creux dans une composante de mouvement vertical ou antérieur.

13. Système (1) pour déterminer un mouvement d'ambulation d'un sujet, le système comprenant :

un accéléromètre (5) pour l'acquisition de données d'accéléromètre indiquant un mouvement du tronc du sujet ; et
l'appareil de traitement (10) pour déterminer un mouvement d'ambulation du sujet selon la revendication 1.

14. Procédé (20) réalisé par un appareil de traitement (10) pour déterminer un mouvement d'ambulation d'un sujet, le procédé comprenant les étapes consistant à
obtenir des données d'accéléromètre indiquant un mouvement de tronc du sujet (S21) ;
réaliser (S22A) un filtrage passe-haut sur les données d'accéléromètre pour obtenir des données d'accéléromètre filtrées passe-haut ;
extraire au moins l'une parmi une pluralité de composantes de mouvement orthogonal à partir des données d'accéléromètre filtrées passe-haut (S22B) en utilisant une technique de séparation de source ;

réaliser (S22B) un filtrage passe-bas sur les données d'accéléromètre pour obtenir des données d'accéléromètre filtrées passe-bas ;

déterminer une composante de gravité à partir des données d'accéléromètre filtrées passe-bas (S23B) ;

comparer une direction d'au moins l'une desdites composantes de mouvement orthogonal avec une direction de la composante de gravité (S24) ; et

classer un mouvement du sujet en tant qu'ambulation si ladite composante de mouvement est orthogonale ou parallèle à ladite composante de gravité (S25).

15. Programme informatique comprenant des moyens de codage de programme pour entraîner l'exécution par un ordinateur des étapes du procédé revendiquées dans la revendication 14 lorsque ledit programme informatique est exécuté sur l'ordinateur.

FIG.1

FIG.2

FIG.3

FIG.4A

FIG.4B

FIG.5

FIG.6A

FIG.6B

FIG.7A

FIG.7B

FIG.8

EP 3 442 403 B1

FIG.9

FIG.10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140019080 A1
- US 20080190202 A
- US 20150272480 A
- US 20130298636 A
- US 20080082018 A

**Non-patent literature cited in the description**

- **WANG YUN-JIA et al.** *Journal of Chinese Inertial Technology,* 2014, vol. 22 (4), 426-431
- **CHOI et al.** Blind Source Separation and Independent Component Analysis: A Review. *Neural Information Processing - Letters and Reviews,* January 2005, vol. 6 (1